# EUROPEAN PATENT APPLICATION

(11) **EP 4 649 953 A1**
(43) Date of publication of application: **19.11.2025**
(21) Application number: 25173727.6
(22) Date of filing: 30.04.2025
(51) Int. Cl.: A61K 36/899, A61K 31/355, A61K 31/575, A61P 13/08

(54) **RICE BRAN OIL FOR PREVENTING OR TREATING PROSTATIC HYPERPLASIA**

(30) Priority: 07.05.2024 CN 202410557220
(71) Applicant: Jiangsu Hankang Biotechnology Co. Ltd., Yixing Jiangsu 214264 (CN)
(72) Inventor: ZHANG, Jingyi, Yixing, Jiangsu, 214264 (CN)
(74) Representative: Dilg, Haeusler, Schindelmann Patentanwaltsgesellschaft mbH

(57) **Abstract**

The present invention discloses a use of rice bran oil in the manufacture of a medicament for preventing or treating prostatic hyperplasia. The present invention is the first to discover and verify that rice bran oil has the effect of inhibiting benign prostatic hyperplasia and associated lower urinary tract symptoms.

## Description

### TECHNICAL FIELD

The present disclosure relates to a use of rice bran oil in the manufacture of a medicament for preventing or treating prostatic hyperplasia, and specifically relates to a use of rice bran oil in the manufacture of a medicament for preventing or treating benign prostatic hyperplasia and associated lower urinary tract symptoms.

### BACKGROUND

Benign prostatic hyperplasia (BPH) is a noncancerous (benign) enlargement of the prostate that may cause dysuria and is also a common and frequently occurring disease in elderly men. Epidemiological research data indicate that the incidence of BPH increases with age, particularly after the age of 50, but the precise etiology remains unclear. In cases of BPH, approximately 50% or more of patients experience bladder outlet obstruction (BOO), including symptoms such as urinary frequency, urinary urgency, and urge urinary incontinence.

Although clinical treatments for BPH include surgical and pharmacological interventions, pharmacological therapy has become the preferred choice for BPH patients due to their generally advanced age. Alpha-adrenergic receptor blockers (e.g., terazosin, doxazosin, tamsulosin, alfuzosin, or silodosin) can relax certain prostate and bladder neck muscles while also potentially improving urine flow. Some medications (e.g., finasteride and dutasteride) may block the effects of male hormones responsible for prostate growth, causing the prostate to shrink and preventing or delaying the need for surgery or other treatments. However, it may take 3 months or longer of finasteride and dutasteride use before symptom relief is achieved. There are also some patients who do not experience relief from their symptoms after taking this type of medication. Some males undergo treatment with alpha-adrenergic receptor blockers combined with finasteride or dutasteride. Saw palmetto exhibits antibacterial and anti-inflammatory, expectorant and antiasthmatic, sedative, antispasmodic, endocrine regulation, diuretic, appetite stimulation, and anti-tumor effects. Saw palmetto extract has the function of inhibiting 5α-reductase activity, reducing dihydrotestosterone production, and antagonizing the binding of androgens to androgen receptors in prostate tissue. In addition, the saw palmetto extract exhibits adrenergic antagonistic effects and calcium-blocking effects, thereby improving bladder function and exerting antispasmodic effects. Therefore, the saw palmetto extract is a highly effective natural therapeutic agent for BPH (or symptoms of prostate enlargement caused by BPH).

It is generally understood that rice bran oil is a by-product obtained from rice processing, which is extracted from crude rice bran oil through direct pressing or solvent extraction, and then refined into finished rice bran oil with high nutritional value. Rice bran oil is a highly nutritious vegetable oil with an absorption rate of 90% or more after consumption. The fatty acids, vitamin E, sterols, oryzanol, and other components in rice bran oil are beneficial for human absorption, and possess advantageous effects such as removing cholesterol from the blood, lowering blood lipids, and promoting human growth and development. Oryzanol is a family of compounds composed of more than a dozen sterol ferulates, which can inhibit autologous cholesterol synthesis, reduce serum cholesterol concentration, promote blood circulation, regulate endocrine and autonomic nervous functions, and facilitate growth and development in humans and animals. Oryzanol promotes microvascular circulation in the skin, protects the skin, and is also effective against conditions such as concussions. Rice bran oil also contains a large amount of nutrients such as fat-soluble vitamins, sitosterol, and other phytosterols from rice bran and germ. However, there have been no reports on the use of rice bran oil in the treatment of BPH.

### SUMMARY

The present disclosure provides a use of rice bran oil in the manufacture of a medicament for preventing or treating benign prostatic hyperplasia. The present disclosure first discovers that rice bran oil can improve urinary parameters, reduce prostate volume, and exert inhibitory effects on benign prostatic hyperplasia and associated lower urinary tract symptoms.

To achieve the objectives as described above, the present disclosure adopts the following technical solutions:

**The present disclosure provides a use of rice bran oil in the manufacture of a medicament for preventing or treating benign prostatic hyperplasia.**

In the present disclosure, the rice bran oil refers to refined rice bran oil or a redissolved product of refined rice bran oil; wherein the refined rice bran oil is processed from crude rice bran oil or a redissolved product of crude rice bran oil, and the redissolved product of refined rice bran oil is prepared by using refined rice bran oil as a base oil and redissolving other substances.

In some embodiments, the rice bran oil is refined rice bran oil, which is prepared by refining crude rice bran oil or a redissolved product of crude rice bran oil, meeting the quality standards (including, but not limited to, GB/T 19112, any enterprise quality standards, or any industry quality standards) and hygienic requirements (including, but not limited to, GB 2716 or GB 2760) for refined oil and being directly suitable for human consumption.

In some embodiments, the rice bran oil is a redissolved product of refined rice bran oil, which is prepared by using refined rice bran oil as a base oil and redissolving other substances; wherein the refined rice bran oil is prepared by refining crude rice bran oil or a redissolved product of crude rice bran oil, meeting the quality standards (including, but not limited to, GB/T 19112, any enterprise quality standards, or any industry quality standards) and hygienic requirements (including, but not limited to, GB 2716 or GB 2760) for refined oil and being directly suitable for human consumption.

In some embodiments, the other substances redissolved in the redissolved product of refined rice bran oil are oryzanol, vitamin E, and phytosterols.

In the present disclosure, the crude rice bran oil may be rice bran oil prepared by direct pressing or solvent extraction from rice bran, which has not undergone any physical, chemical, or biological refining processes and is not directly suitable for human consumption. Herein, the rice bran is obtained from rice processing.

In the present disclosure, the redissolved product of crude rice bran oil may be prepared by using crude rice bran oil as a base oil and redissolving other substances. Herein, the other substances are preferably oryzanol, vitamin E, and phytosterols.

In the present disclosure, the refining process may include a physical, chemical, or biological refining process.

In some embodiments, the rice bran oil is refined rice bran oil obtained by subjecting crude rice bran oil to a physical refining process known in the art, such as Shengfukang rice bran oil.

In some embodiments, the rice bran oil comprises oryzanol, vitamin E, and phytosterols.

In some embodiments, the mass ratio of the oryzanol, vitamin E, and phytosterols in the rice bran oil is (0.1 to 25):1:(1 to 30), preferably (10 to 15):1:(12.5 to 17.5), for example, 15:1:17.5.

In some embodiments, the content of the oryzanol in the rice bran oil is 5000 mg/kg or more, preferably 19000 to 20000 mg/kg.

In some embodiments, the content of the vitamin E in the rice bran oil is 100 mg/kg or more, preferably 800 to 1200 mg/kg.

In some embodiments, the content of the phytosterols in the rice bran oil is 5000 mg/kg or more, preferably 20000 to 22000 mg/kg.

In some embodiments, the phytosterols comprise sitosterol, wherein the content of the sitosterol is preferably 40 to 55%.

In some embodiments, the daily dose of the rice bran oil is 10 to 40 g. The daily dose may be calculated based on the dose conversion standard between mice and humans.

In some embodiments, the symptoms of benign prostatic hyperplasia include benign prostatic hyperplasia and associated lower urinary tract symptoms; wherein the associated lower urinary tract symptoms include, for example, one or more of urinary frequency, urinary urgency, urge urinary incontinence, and dysuria.

In some embodiments, the use achieves prevention or treatment of benign prostatic hyperplasia by reducing dihydrotestosterone (DHT) levels, inhibiting glandular epithelial cell hyperplasia, or inhibiting stromal tissue hyperplasia.

In some embodiments, the dosage form of the medicament comprises tablets and capsules.

In some embodiments, the medicament further comprises an excipient, wherein the excipient comprises one or more of a binder, a disintegrant, a lubricant, and a filler.

In some embodiments, the binder is selected from one or more of starch, sodium carboxymethyl cellulose, hydroxypropyl methylcellulose, hydroxypropyl cellulose, methyl cellulose, ethyl cellulose, povidone, gelatin, and polyethylene glycol.

In some embodiments, the disintegrant is selected from one or more of starch, sodium carboxymethyl starch, low-substituted hydroxypropyl cellulose, crospovidone, and croscarmellose sodium.

In some embodiments, the lubricant is selected from one or more of magnesium stearate, colloidal silicon dioxide, talc, polyethylene glycol, and magnesium lauryl sulfate.

In some embodiments, the filler is selected from one or more of starch, sucrose, dextrin, lactose, microcrystalline cellulose, inorganic salts, and mannitol.

**The present disclosure further provides a method for preventing or treating benign prostatic hyperplasia, comprising administering rice bran oil to a patient.**

In some embodiments, the rice bran oil is as defined above.

In some embodiments, the benign prostatic hyperplasia is as defined above.

In some embodiments, the daily dose of the rice bran oil is 10 to 40 g. The daily dose may be calculated based on the dose conversion standard between mice and humans.

**The present disclosure further provides a rice bran oil for preventing or treating benign prostatic hyperplasia.**

In some embodiments, the benign prostatic hyperplasia is as defined above.

**The present disclosure further provides a medicament for preventing or treating benign prostatic hyperplasia, which is prepared using rice bran oil.**

In some embodiments, the rice bran oil is as defined above.

In some embodiments, the benign prostatic hyperplasia is as defined above.

**The present disclosure further provides a use of rice bran oil in the manufacture of a dihydrotestosterone inhibitor.**

In some embodiments, the rice bran oil is as defined above.

**The present disclosure further provides a use of rice bran oil in the manufacture of an inhibitor for glandular epithelial cell hyperplasia.**

In some embodiments, the rice bran oil is as defined above.

**The present disclosure further provides a use of rice bran oil in the manufacture of an inhibitor for stromal tissue hyperplasia.**

In some embodiments, the rice bran oil is as defined above.

**The present disclosure further provides a use of rice bran oil in a food or health product, wherein the food or health product has the effect of preventing or treating benign prostatic hyperplasia.**

In some embodiments, the rice bran oil is as defined above.

In some embodiments, the benign prostatic hyperplasia is as defined above.

Based on common knowledge in the art, the aforementioned preferred conditions can be combined in any manner to obtain various preferred embodiments of the present disclosure.

The reagents and raw materials used in the present disclosure are all commercially available.

The positive and progressive effects of the present disclosure are as follows:

The present disclosure is the first to discover and verify that rice bran oil has the effect of inhibiting benign prostatic hyperplasia and associated lower urinary tract symptoms, with efficacy superior to that of saw palmetto extract, a highly effective natural therapeutic agent for BPH (or symptoms of prostate enlargement caused by BPH). Preferably, the rice bran oil comprises oryzanol, vitamin E, and phytosterols within a specific range of concentrations and ratios. These three active ingredients exhibit a synergistic effect in combination with the remaining components of rice bran oil.

### BRIEF DESCRIPTION OF THE DRAWINGS

FIG. 1 shows the prostate morphology of mice in different groups in Example 2.
FIG. 2 shows the Masson staining results of the prostate of mice in different groups in Example 6.

### DETAILED DESCRIPTION OF THE PREFERRED EMBODIMENT

The following examples provide further illustration of the present disclosure, but the scope of the present disclosure is not limited to these examples. Those skilled in the art will appreciate that various changes and modifications can be made to the present disclosure without departing from the spirit and scope of the present disclosure. The present disclosure provides a general and/or specific description of the materials and experimental methods used in the tests. Although many materials and operational methods used to achieve the objectives of the present disclosure are well-known in the art, the present disclosure still describes them herein in as much detail as possible.

In the following examples, experimental methods without specified conditions were selected according to conventional methods and conditions, or according to instructions provided by the manufacturer.

The test sample, Shengfukang rice bran oil, was provided by Jiangsu Hankang Biotechnology Co., Ltd., containing 19,000 to 20,000 ppm of oryzanol, 20,000 to 22,000 ppm of phytosterols (of which sitosterol accounted for 40 to 55%), and 800 to 1,200 ppm of vitamin E.

Saw palmetto extract was provided by TAD Pharma GmbH, Germany, with glycerol as the solvent.

Testosterone propionate injection was purchased from Shanghai General Pharmaceutical Co., Ltd.; specification: 1 mL, 25 mg, 10 vials per package.

Dihydrotestosterone (DHT) ELISA kit was purchased from R&D Systems, USA, Lot No.: 20120801A.

Experimental results are expressed as mean ± standard deviation. Parametric or non-parametric variance analysis was performed, with p < 0.05 considered statistically significant and p < 0.01 considered highly statistically significant.

The recommended daily dose of rice bran oil for humans in the present disclosure was derived from the doses used in experimental animals in the examples, as detailed in Table 1:

**Table 1. Dose conversion coefficient between animals and humans per kg body weight**

| Conversion coefficient W | | Group A of animals or adults | | | | | | |
|---|---|---|---|---|---|---|---|---|
| | | Mouse 0.02 kg | Rat 0.2 kg | Guinea pig 0.4 kg | Rabbit 1.5 kg | Cat 2 kg | Dog 12 kg | Adult 60 kg |
| Group B of animals or adults | Mouse 20 g | 1.0 | 1.6 | 1.6 | 2.7 | 3.2 | 4.8 | 9.01 |
| | Rat 0.2 kg | 0.7 | 1.0 | 1.14 | 1.88 | 2.3 | 3.6 | 6.25 |
| | Guinea pig 0.4 kg | 0.61 | 0.87 | 1.0 | 1.65 | 2.05 | 3.0 | 5.55 |
| | Rabbit 1.5 kg | 0.37 | 0.52 | 0.6 | 1.0 | 1.23 | 1.76 | 2.30 |
| | Cat 2.0 kg | 0.30 | 0.42 | 0.48 | 0.81 | 1.0 | 1.44 | 2.70 |
| | Dog 12 kg | 0.21 | 0.28 | 0.34 | 0.56 | 0.68 | 1.0 | 1.88 |
| | Adult 60 kg | 0.11 | 0.16 | 0.18 | 0.304 | 0.371 | 0.531 | 1.0 |

For example, based on the dose per kg body weight in mice, the dose per kg body weight in humans was calculated using the following formula:

Dose per kg body weight in humans = Dose per kg body weight in mice × conversion coefficient of 0.11.

### Example 1: Method for establishing an animal model of prostatic hyperplasia

### 1. Modeling method:

Non-castrated mouse model (model group): A prostatic hyperplasia model was induced by intraperitoneal injection of testosterone propionate at a dose of 5 mg/kg for 31 consecutive days.

### 2. Detection indicators:

24-hour urine volume of the mice was measured 24 hours before tissue collection.

During sampling, the mice were weighed, and blood was collected from the orbital sinus. Serum was separated by centrifugation for measurement of dihydrotestosterone (DHT) levels. The mice were then euthanized by cervical dislocation, and prostate tissue was rapidly excised. The prostate wet weight was measured, and the prostate index was calculated (prostate index = prostate wet weight in mg / mouse body weight in g). The prostate tissue was fixed in 10% formaldehyde solution, embedded in paraffin, sectioned, and stained with hematoxylin-eosin (HE). Morphological changes in the prostate tissue of each group were observed under a light microscope.

### 3. Pathological scoring:

"-" indicates the prostate tissue being essentially normal, with no hyperplasia observed in the glands, glandular epithelial cells, or stroma; "+" indicates hyperplasia of some glandular epithelium, presenting as pseudopapillary structures, with the stroma being essentially normal or showing slight hyperplasia of fibrous connective tissue; "++" indicates mild hyperplasia of the prostate glands, with mild hyperplasia also observed in the glandular epithelium as well as stroma and smooth muscle, presenting as pseudopapillary structures; "+++" indicates significant hyperplasia of the prostate glands and some glandular epithelium, with numerous papillary or pseudopapillary protrusions, as well as significant hyperplasia of the stroma and smooth muscle.

As shown in Table 2, the prostate wet weight, prostate index, DHT levels, and 24-hour urine volume were significantly increased in the model group of mice. As shown in Table 3, the model group of mice exhibited significant prostatic hyperplasia, indicating the successful establishment of the mouse model of prostatic hyperplasia prepared by the method of the present disclosure.

**Table 2. Detection results of mouse model of prostatic hyperplasia**

| | Prostate wet weight (mg ± SD) | Prostate index | DHT levels (nmol/L ± SD) | 24-hour urine volume (mL ± SD) |
|---|---|---|---|---|
| Control | 61.53 ± 5.29 | 2.03 ± 0.18 | 4.27 ± 1.32 | 0.52 ± 0.09 |
| Model | 93.66 ± 11.82*** | 3.88 ± 0.44*** | 9.07 ± 2.28*** | 1.63 ± 0.27*** |

| | | | | |
|---|---|---|---|---|
| *** indicates P < 0.001 compared to the control group. | | | | |

**Table 3. Pathological scoring of prostate in mouse model of prostatic hyperplasia**

| Group | Number of mice | - | + | ++ | +++ |
|---|---|---|---|---|---|
| Control | 12 | 12 | 0 | 0 | 0 |
| Model | 12 | 0 | 0 | 2 | 10 |

### Example 2: Effect of rice bran oil on prostate wet weight in mice with prostatic hyperplasia

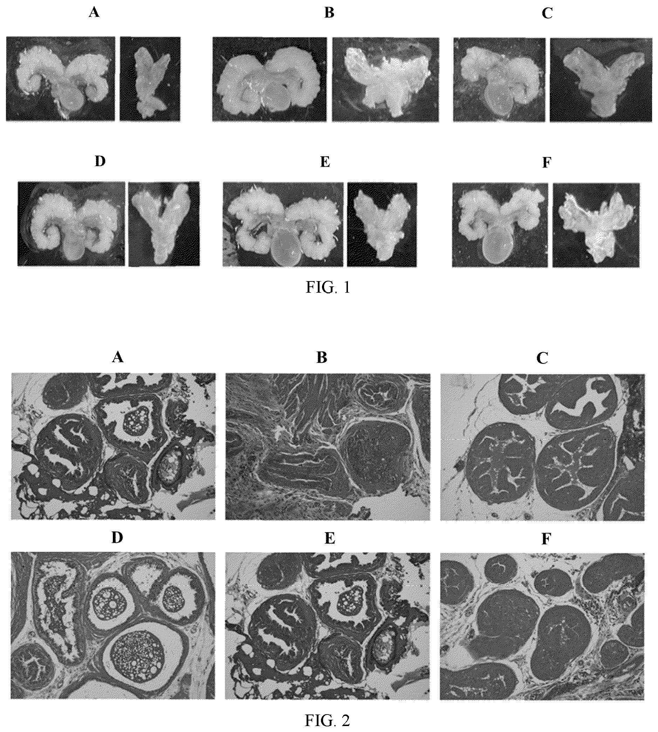

A mouse model of prostatic hyperplasia was established according to the method in Example 1, and prostate wet weight was measured. Rice bran oil was administered concurrently during the modeling period, with a dosing cycle of 31 days. The specific groups are shown in Table 4. The results are shown in FIG. 1 and Table 5. As shown in FIG. 1, compared to the control group (A in FIG. 1), the model group (B in FIG. 1) of mice showed a significant increase in prostate volume. Compared to the model group, groups of saw palmetto extract (C in FIG. 1) and rice bran oil at various doses (D, E, and F in FIG. 1) all showed a decrease in prostate volume in the mouse model of prostatic hyperplasia. As shown in Table 5, the model group showed a significant increase in prostate wet weight, and saw palmetto extract significantly reduced prostate wet weight in mice. Rice bran oil dose-dependently reduced prostate wet weight in mice, and the high-dose and medium-dose rice bran oil groups exhibited superior efficacy in reducing prostate wet weight compared to the saw palmetto extract group.

**Table 4. Administration regimen of rice bran oil in different groups**

| No. | Group | Daily dose per kg body weight |
|---|---|---|
| A | Control | Glycerol |
| B | Model | Glycerol |
| C | Positive control | Saw palmetto extract 100 mg/kg/day |
| D | High-dose rice bran oil | Rice bran oil 4.5 mL/kg/day (4.14 g/kg/day) |
| E | Medium-dose rice bran oil | Rice bran oil 3 mL/kg/day (2.76 g/kg/day) |
| F | Low-dose rice bran oil | Rice bran oil 1.5 mL/kg/day (1.38 g/kg/day) |

The density of rice bran oil is 0.92 g/mL.

**Table 5. Effect of rice bran oil on prostate wet weight in mice with prostatic hyperplasia**

| Group | Control A | Model B | Positive control C | High-dose rice bran oil D | Medium-dose rice bran oil E | Low-dose rice bran oil F |
|---|---|---|---|---|---|---|
| Prostate wet weight (mg ± SD) | 58.31 ± 4.27 | 97.90 ± 11.33*** | 79.28 ± 6.69^{#} | 68.37 ± 5.98^{##} | 74.32 ± 8.04^{#} | 82.38 ± 7.71 |

| | | | | | | |
|---|---|---|---|---|---|---|
| *** indicates P < 0.001 compared to the control group; # indicates P < 0.05 compared to the model group; ## indicates P < 0.01 compared to the model group. | | | | | | |

### Example 3: Effect of rice bran oil on prostate index in mice with prostatic hyperplasia

A mouse model of prostatic hyperplasia was established according to the method in Example 1, and prostate index was measured. Rice bran oil was administered concurrently during the modeling period, with a dosing cycle of 31 days. The specific groups are shown in Table 4. The results are shown in Table 6. As shown in Table 6, the model group showed a significant increase in prostate index, and saw palmetto extract significantly reduced prostate index in mice. Rice bran oil dose-dependently reduced prostate index in mice, and the high-dose rice bran oil group exhibited superior efficacy in reducing prostate index compared to the saw palmetto extract group.

**Table 6. Effect of rice bran oil on prostate index in mice with prostatic hyperplasia**

| Group | Control A | Model B | Positive control C | High-dose rice bran oil D | Medium-dose rice bran oil E | Low-dose rice bran oil F |
|---|---|---|---|---|---|---|
| Prostate index | 2.24 ± 0.37 | 4.01 ± 0.68*** | 3.07 ± 0.51^{#} | 2.62 ± 0.33^{##} | 3.09 ± 0.43^{#} | 3.11 ± 0.57^{#} |

| | | | | | | |
|---|---|---|---|---|---|---|
| *** indicates P < 0.001 compared to the control group; # indicates P < 0.05 compared to the model group; ## indicates P < 0.01 compared to the model group. | | | | | | |

### Example 4: Effect of rice bran oil on DHT in mice with prostatic hyperplasia

A mouse model of prostatic hyperplasia was established according to the method in Example 1, and DHT levels in serum were measured. Rice bran oil was administered concurrently during the modeling period, with a dosing cycle of 31 days. The specific groups are shown in Table 4. The results are shown in Table 7. As shown in Table 7, the model group showed a significant increase in DHT levels in serum, and saw palmetto extract significantly reduced DHT levels in mice. Rice bran oil dose-dependently reduced DHT levels in mice, and the low-dose, medium-dose, and high-dose rice bran oil groups all exhibited superior efficacy in reducing DHT levels compared to the saw palmetto extract group.

**Table 7. Effect of rice bran oil on DHT in mice with prostatic hyperplasia**

| Group | Control A | Model B | Positive control C | High-dose rice bran oil D | Medium-dose rice bran oil E | Low-dose rice bran oil F |
|---|---|---|---|---|---|---|
| DHT (nmol/L ± SD) | 4.07 ± 0.62 | 9.95 ± 2.26*** | 7.69 ± 2.09^{#} | 6.22 ± 1.83^{##} | 6.62 ± 0.91^{##} | 7.35 ± 2.41^{#} |

| | | | | | | |
|---|---|---|---|---|---|---|
| *** indicates P < 0.001 compared to the control group; # indicates P < 0.05 compared to the model group; ## indicates P < 0.01 compared to the model group. | | | | | | |

### Example 5: Effect of rice bran oil on 24-hour urine volume in mice with prostatic hyperplasia

A mouse model of prostatic hyperplasia was established according to the method in Example 1, and 24-hour urine volume was measured. Rice bran oil was administered concurrently during the modeling period, with a dosing cycle of 31 days. The specific groups are shown in Table 4. The results are shown in Table 8. As shown in Table 8, the model group showed a significant increase in 24-hour urine volume, and saw palmetto extract significantly reduced 24-hour urine volume in mice. Rice bran oil dose-dependently reduced 24-hour urine volume in mice, and the high-dose rice bran oil group exhibited superior efficacy in reducing 24-hour urine volume compared to the saw palmetto extract group.

**Table 8. Effect of rice bran oil on 24-hour urine volume in mice with prostatic hyperplasia**

| Group | Control A | Model B | Positive control C | High-dose rice bran oil D | Medium-dose rice bran oil E | Low-dose rice bran oil F |
|---|---|---|---|---|---|---|
| 24-hour urine volume (mL ± SD) | 0.55 ± 0.07 | 1.52 ± 0.29*** | 1.01 ± 0.21^{#} | 0.82 ± 0.18^{##} | 1.05 ± 0.33^{#} | 1.14 ± 0.23 |

| | | | | | | |
|---|---|---|---|---|---|---|
| *** indicates P < 0.001 compared to the control group; # indicates P < 0.05 compared to the model group; ## indicates P < 0.01 compared to the model group. | | | | | | |

### Example 6: Effect of rice bran oil on pathological scoring of prostate in mice with prostatic hyperplasia

A mouse model of prostatic hyperplasia was established according to the method in Example 1, and pathological scoring of prostate was measured. Rice bran oil was administered concurrently during the modeling period, with a dosing cycle of 31 days. The specific groups are shown in Table 4. The results are shown in FIG. 2 and Table 9. As shown in FIG. 2 and Table 9, the control group (A in FIG. 2) of mice showed essentially normal prostate glands and stromal tissues, with no hyperplasia observed. The model group (B in FIG. 2) of mice showed significant hyperplasia in the prostate glands and some glandular epithelial cells, with hyperplasia observed in stromal tissues and significant fibrotic changes. The saw palmetto extract group (C in FIG. 2) of mice showed hyperplasia in the prostate glands, with significant hyperplasia of glandular epithelium and slight hyperplasia of stroma. The high-dose rice bran oil group (D in FIG. 2) of mice showed essentially normal prostate glands, with significantly inhibited hyperplasia of glandular epithelium and essentially normal stroma. The medium-dose rice bran oil group (E in FIG. 2) of mice showed mild hyperplasia in the prostate glands, with significant hyperplasia of glandular epithelium and essentially normal stroma. The low-dose rice bran oil group (F in FIG. 2) of mice showed hyperplasia in the prostate glands, with slight hyperplasia of glandular epithelial cells and essentially normal stroma.

**Table 9. Effect of rice bran oil on pathological scoring in mice with prostatic hyperplasia**

| Group | Control A | Model B | Positive control C | High-dose rice bran oil D | Medium-dose rice bran oil E | Low-dose rice bran oil F |
|---|---|---|---|---|---|---|
| Number of mice | 12 | 12 | 12 | 12 | 12 | 12 |
| - | 12 | 0 | 0 | 1 | 0 | 0 |
| + | 0 | 0 | 2 | 7 | 5 | 3 |
| ++ | 0 | 1 | 4 | 4 | 6 | 9 |
| +++ | 0 | 11 | 6 | 0 | 1 | 0 |

The meanings of pathological scores "-", " +", " ""++", and "+++" in the table refer to Example 1.

## Claims

1. Rice bran oil for use as a medicament for preventing or treating benign prostatic hyperplasia.

2. The rice bran oil for use according to claim 1, wherein the rice bran oil comprises oryzanol, vitamin E, and phytosterols.

3. The rice bran oil for use according to claim 2, wherein the mass ratio of the oryzanol, vitamin E, and phytosterols in the rice bran oil is (0.1 to 25):1:(1 to 30), preferably (10 to 15):1:(12.5 to 17.5), for example, 15:1:17.5.

4. The rice bran oil for use according to claim 2, wherein the content of the oryzanol in the rice bran oil is 5000 mg/kg or more, preferably 19000 to 20000 mg/kg;
and/or, the content of the vitamin E in the rice bran oil is 100 mg/kg or more, preferably 800 to 1200 mg/kg;
and/or, the content of the phytosterols in the rice bran oil is 5000 mg/kg or more, preferably 20000 to 22000 mg/kg;
and/or, the phytosterols comprise sitosterol, wherein the content of the sitosterol is preferably 40 to 55%.

5. The rice bran oil for use according to claim 1, wherein the rice bran oil is refined rice bran oil, which is prepared by refining crude rice bran oil or a redissolved product of crude rice bran oil, meeting the quality standards and hygienic requirements for refined oil and being directly suitable for human consumption;
or, the rice bran oil is a redissolved product of refined rice bran oil, which is prepared by using refined rice bran oil as a base oil and redissolving other substances; wherein the refined rice bran oil is prepared by refining crude rice bran oil or a redissolved product of crude rice bran oil, meeting the quality standards and hygienic requirements for refined oil and being directly suitable for human consumption.

6. The rice bran oil for use according to claim 1, wherein the symptoms of benign prostatic hyperplasia comprise benign prostatic hyperplasia and associated lower urinary tract symptoms; wherein the associated lower urinary tract symptoms comprise, for example, one or more of urinary frequency, urinary urgency, urge urinary incontinence, and dysuria.

7. The rice bran oil for use according to claim 1, wherein the rice bran oil achieves prevention or treatment of benign prostatic hyperplasia by reducing dihydrotestosterone levels, inhibiting glandular epithelial cell hyperplasia, or inhibiting stromal tissue hyperplasia.

8. An inhibitor comprising rice bran oil, wherein the inhibitor is a dihydrotestosterone inhibitor, an inhibitor for glandular epithelial cell hyperplasia, or an inhibitor for stromal tissue hyperplasia.

9. The inhibitor according to claim 8, wherein the rice bran oil comprises oryzanol, vitamin E, and phytosterols.

10. The inhibitor according to claim 9, wherein the mass ratio of the oryzanol, vitamin E, and phytosterols in the rice bran oil is (0.1 to 25):1:(1 to 30), preferably (10 to 15):1:(12.5 to 17.5), for example, 15:1:17.5.

11. The inhibitor according to claim 9, wherein the content of the oryzanol in the rice bran oil is 5000 mg/kg or more, preferably 19000 to 20000 mg/kg;
and/or, the content of the vitamin E in the rice bran oil is 100 mg/kg or more, preferably 800 to 1200 mg/kg;
and/or, the content of the phytosterols in the rice bran oil is 5000 mg/kg or more, preferably 20000 to 22000 mg/kg;
and/or, the phytosterols comprise sitosterol, wherein the content of the sitosterol is preferably 40 to 55%.

12. The inhibitor according to claim 8, wherein the rice bran oil is refined rice bran oil, which is prepared by refining crude rice bran oil or a redissolved product of crude rice bran oil, meeting the quality standards and hygienic requirements for refined oil and being directly suitable for human consumption;
or, the rice bran oil is a redissolved product of refined rice bran oil, which is prepared by using refined rice bran oil as a base oil and redissolving other substances; wherein the refined rice bran oil is prepared by refining crude rice bran oil or a redissolved product of crude rice bran oil, meeting the quality standards and hygienic requirements for refined oil and being directly suitable for human consumption.

13. The inhibitor according to claim 8, wherein the symptoms of benign prostatic hyperplasia comprise benign prostatic hyperplasia and associated lower urinary tract symptoms; wherein the associated lower urinary tract symptoms comprise, for example, one or more of urinary frequency, urinary urgency, urge urinary incontinence, and dysuria.
